(19) Europäisches Patentamt / European Patent Office / Office européen des brevets

(11) **EP 4 085 836 A1**

(12) **EUROPEAN PATENT APPLICATION**

(43) Date of publication:
**09.11.2022  Bulletin 2022/45**

(21) Application number: **21172330.9**

(22) Date of filing: **05.05.2021**

(51) International Patent Classification (IPC):
***A61B 5/145*** $^{(2006.01)}$

(52) Cooperative Patent Classification (CPC):
**A61B 5/14521; A61B 5/14532; A61B 5/14546**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **VAN LIESHOUT, Ron Martinus Laurentius
5656 AE Eindhoven (NL)**
• **DELLIMORE, Kiran Hamilton J.
5656 AE Eindhoven (NL)**
• **PELSSERS, Eduard Gerard Marie
5656 AE Eindhoven (NL)**
• **JOHNSON, Mark Thomas
5656 AE Eindhoven (NL)**

(74) Representative: **Philips Intellectual Property &
Standards
High Tech Campus 52
5656 AG Eindhoven (NL)**

(54) **DETERMINING THE CONCENTRATION OF A BIOMARKER IN BLOOD OF A SUBJECT**

(57)    According to an aspect, there is provided a method (200, 400) for measuring a concentration of a biomarker in sweat generated by a subject, the method comprising: applying (202) a negative pressure to the subject at the location of the sweat gland; measuring (204), after a defined duration of the negative pressure being applied, a concentration of the biomarker in sweat secreted from the sweat gland of the subject.

200

202

204

Fig. 3

EP 4 085 836 A1

**Description**

FIELD OF THE INVENTION

The invention relates to determining the concentration of a biomarker in blood of a subject and, more particularly, to determining the concentration of a biomarker in a subject's blood based on a measurement of the concentration of the biomarker in sweat of the subject.

BACKGROUND OF THE INVENTION

Non-invasive, continuous and prolonged monitoring of biomarkers that indicate health and well-being is useful for example for monitoring dehydration, stress, sleep, children's health and for perioperative monitoring. Sweat is a non-obtrusively accessible bio-fluid containing physiologically and metabolically rich information. Some examples of clinically relevant components of sweat are sodium ions, chlorine ions and potassium ions to monitor dehydration, lactate as an early warning for inflammation (relevant to sepsis), glucose for diabetes and neonates and cortisol for sleep and stress monitoring. Therefore, analysing components in sweat can provide useful medical information about a person.

However, the correlation between concentrations of components in blood and sweat is lacking for some biomarkers. In other words, the concentration of a biomarker in sweat of a person may not accurately represent the concentration of the biomarker in the person's blood.

SUMMARY OF THE INVENTION

There is a desire to be able to take account of the difference between the concentration of a component (such as a biomarker) in sweat and its concentration in blood, and take action to mitigate the difference, such that a measurement of the component's concentration in sweat can be taken as an accurate representation of the component's concentration in blood.

The inventors have recognised that various mechanisms that occur within the human body affect the concentration of biomarkers measurable in sweat. One such mechanism involves the movement of fluids between capillary beds and interstitial fluid and cells surrounding blood capillaries, sometimes referred to as capillary exchange, and the movement of fluids between sweat ducts of sweat glands and tissues (e.g. interstitial fluid and cells) surrounding the sweat ducts. According to various embodiments disclosed herein, measurements of the concentration of a biomarker are taken after 'negative' pressure has been applied to the person. As used herein, a negative pressure is intended to refer to a pressure below the ambient air pressure. A negative pressure may be referred to as an under-pressure, and/or may be said to be vacuum assisted. In some examples, a negative pressure may be created by using a suction pump or vacuum pump, for example by reducing the pressure in the region above the subject's skin, as described herein. As a consequence of applying the negative pressure, a concentration of the biomarker in the sweat corresponds more closely to the concentration of the biomarker in the person's blood, so a good approximation of the concentration of the biomarker in the person's blood can be achieved by analysing the sweat, without the need for intrusive analysis of the subject's blood.

The invention is defined by the independent claims, while dependent claims provide advantageous embodiments.

According to a first specific aspect, there is provided a method for measuring a concentration of a biomarker in sweat generated by a subject, the method comprising: applying a negative pressure to the subject at the location of the sweat gland; measuring a concentration of the biomarker in sweat secreted from the sweat gland of the subject, after the negative pressure has being applied, or while the negative pressure is applied, for a defined duration..

In some embodiments, the applied negative pressure may meet or exceed a defined pressure threshold at which reabsorption of fluid from a sweat duct of the sweat gland into surrounding tissues reduces. In some examples, the applied negative pressure may be such that reabsorption of fluid from the sweat duct into the surrounding tissues reduces to below a defined threshold amount.

Applying a negative pressure may comprise applying a pressure having a magnitude such that the applied negative pressure and an osmotic pressure within tissues surrounding a sweat duct of the sweat gland together exceed a pressure of fluids within the sweat duct of the sweat gland.

In some embodiments, the method may further comprise determining a pressure of fluids within a sweat duct of the sweat gland. The negative pressure applied to the subject may be based on the determined pressure.

In some embodiments, the method may further comprise measuring, prior to applying the negative pressure to the subject, a first sweat rate of the subject. The method may further comprise measuring, following the application of the negative pressure, a second sweat rate of the subject. The method may comprise calculating a concentration of the biomarker in sweat secreted from the sweat gland of the subject based on the measured concentration and on the measured first sweat rate and the measured second sweat rate.

In some embodiments, the method may further comprise applying, after measuring the concentration of the biomarker, a positive pressure to the subject at the location of the sweat gland, so as to increase a pressure in interstitial fluid around the sweat gland.

The method may, in some embodiments, further comprise measuring, during the application of the negative pressure, a plurality of concentrations of the biomarker. The method may further comprise calculating a concentration of the biomarker in sweat secreted from the sweat gland of the subject based on the plurality of measurements.

Applying the negative pressure may comprise decreasing a magnitude of the applied negative pressure in regular steps, and measuring, at each step, a concentration of the biomarker in sweat secreted from the sweat gland of the subject. The method may further comprise generating a calibration curve using the measured concentrations, and calculating a concentration of the biomarker in sweat based on the generated calibration curve.

In some embodiments, the method may further comprise removing, after the expiry of the defined duration, the application of the negative pressure.

According to a second specific aspect, there is provided an apparatus for measuring a concentration of a biomarker in sweat generated by a subject, the apparatus comprising: a biomarker sensor for receiving a volume of sweat secreted from a sweat gland of the subject and measuring a concentration of a biomarker in the volume of sweat; a pressure application unit configured to apply a negative pressure to the subject at the location of the sweat gland; and a processor in communication with the biomarker sensor and the pressure application unit, and configured to: operate the pressure application unit to apply a negative pressure to the subject at the location of the sweat gland; operate the biomarker sensor to measure a concentration of the biomarker in a volume of sweat secreted from the sweat gland after the pressure application unit has applied, or while the pressure application unit applies a negative pressure to the subject, for a defined period .

In some embodiments, the processor may be configured to operate the pressure application unit to apply a negative pressure having a magnitude that meets or exceeds a defined pressure threshold at which reabsorption of fluid from a sweat duct of the sweat gland into surrounding tissues reduces. In some examples, the applied negative pressure may be such that reabsorption of fluid from the sweat duct into the surrounding tissues reduces to below a defined threshold amount.

The apparatus may further comprise a pressure measurement unit for measuring a pressure of fluids within a sweat duct of the sweat gland. The processor may be configured to operate the pressure application unit to apply a negative pressure based on the measured pressure. The apparatus may, in some embodiments, further comprise a timer in communication with the processor, and configured to measure at least one of: a duration of application of the applied negative pressure; and the timing of measurements of the biomarker concentrations.

In some embodiments, the biomarker sensor may be configured to measure the concentration, in the volume of sweat, of a biomarker selected from a group comprising: lactate, sodium ions, potassium ions, chlorine ions, urea, ethanol, ammonium, glucose and cortisol.

According to a third specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to: operate the pressure application unit to apply a negative pressure to the subject at the location of the sweat gland; and operate a biomarker sensor to measure a concentration of the biomarker in a volume of sweat secreted from the sweat gland after the pressure application unit has applied, or while the pressure application unit applies a negative pressure to the subject, for a defined period.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a schematic illustration of an example of a pressure application unit on a body part of a subject;
Fig. 2 is a schematic illustration of an example of an apparatus in accordance with various embodiments;
Fig. 3 is a flowchart of an example of a method of measuring a concentration of a biomarker in sweat generated by a subject;
Fig. 4 is a flowchart of a further example of a method of measuring a concentration of a biomarker in sweat generated by a subject;
Fig. 5 is a schematic illustration of an example of a technique for applying a negative pressure;
Fig. 6 is a schematic illustration of a further example of a technique for applying a negative pressure;

Fig. 7 is a schematic illustration of a further example of an apparatus in accordance with various embodiments; and

Fig. 8 is a schematic illustration of an example of a processor in communication with a computer-readable medium.

DETAILED DESCRIPTION OF EMBODIMENTS

Embodiments disclosed herein provide a mechanism by which a measurement of the concentration of a biomarker in a subject's sweat can be correlated with the concentration of the biomarker in the subject's blood, and this is achieved by measuring the concentration of the biomarker in the sweat while a negative pressure is being applied to the subject. Applying a negative pressure to the skin of the subject in the vicinity of the sweat glands from which the subject sweat is generated helps to counteract various effects that take place within the body that affect the concentration of certain biomarkers in the subject's sweat.

Capillary exchange refers to the mass movement of fluids into and out of capillary beds (i.e. the blood capillary system of a person) and involves two pressure-driven mechanisms. A first mechanism occurs when fluid moves from an area of relatively higher pressure in a capillary bed to an area of relatively lower pressure in tissue next to, or adjacent to, the capillary bed via a process known as filtration. A second mechanism occurs when fluid moves from an area of relatively higher pressure in the tissue to an area of relatively lower pressure in the capillary bed via a process known as reabsorption. Two types of pressure interact to drive these movements: hydrostatic pressure and osmotic pressure.

Hydrostatic pressure is the main contributor of fluid transport between the capillaries and tissue, and can be considered to represent the pressure of a fluid enclosed in a space. Blood hydrostatic pressure is caused by blood confined within blood vessels or heart chambers. More specifically, the pressure exerted by blood against the wall of a capillary is referred to as capillary hydrostatic pressure (CHP), or capillary blood pressure. Tissue surrounding capillaries includes interstitial fluid. As fluid exits a capillary and moves into the surrounding tissue, the hydrostatic pressure in the interstitial fluid increases correspondingly, and is referred to as the interstitial fluid hydrostatic pressure (IFHP). Generally, the CHP originating from arterial pathways is considerably higher than the IFHP, because lymphatic vessels act to absorb excess fluid from the tissues. Thus, fluid generally tends to move out of the capillaries and into the interstitial fluid in the process referred to as filtration.

Osmotic pressure (sometimes referred to as oncotic pressure) acts against the hydrostatic pressure, and serves to draw fluid from the interstitial fluid back into the capillaries.

Filtration out of, and reabsorption into, a capillary occurs in accordance with the balance of hydrostatic pressure and osmotic pressure, as described in the Starling hypothesis:

$$Filtration\ force = k[(P_c + \pi_i) - (P_i + \pi_c)] \qquad [1]$$

where k is a permeability coefficient; $P_c$ = hydrostatic (blood) pressure in the capillary; $\pi_i$ = oncotic pressure in the interstitial fluid; $P_i$ = hydrostatic pressure in the interstitial fluid; and $\pi_c$ = oncotic pressure in the capillary (e.g. from plasma proteins). $P_c$ and $P_i$ are "positive pressures" and $\pi_i$ and $\pi_c$ are "negative pressures". Biomolecules, including those which may be detected and used as biomarkers, may pass with fluid from the capillary into the interstitial fluid during the filtration process.

Other processes taking place within the body also contribute to the movement of fluid containing biomarkers. Biomarkers present in sweat within the body can be reabsorbed into capillaries if, for example, the hydrostatic pressure in the interstitial fluid is greater than the hydrostatic pressure in the capillary. Furthermore, biomarkers may be caused to move into the interstitial fluid by the process of perfusion around the sweat gland. Perfusion is a process by which fluid (e.g. fluid containing biomarkers) moves up through a circulatory system to an organ (e.g. skin) or tissue. Movement of fluids through a subject's body can be affected or influenced by the increase of pressure in the subject's tissue and interstitial fluid (e.g. by the application of external pressure on the subject's skin).

Another process that takes place within the subject's body that impacts the correlation between a concentration of a biomarker in the subject's blood and the concentration of the biomarker in the subject's sweat is the movement of fluids between the sweat gland and the tissues (e.g. the interstitial fluid and cells) surrounding the sweat gland.

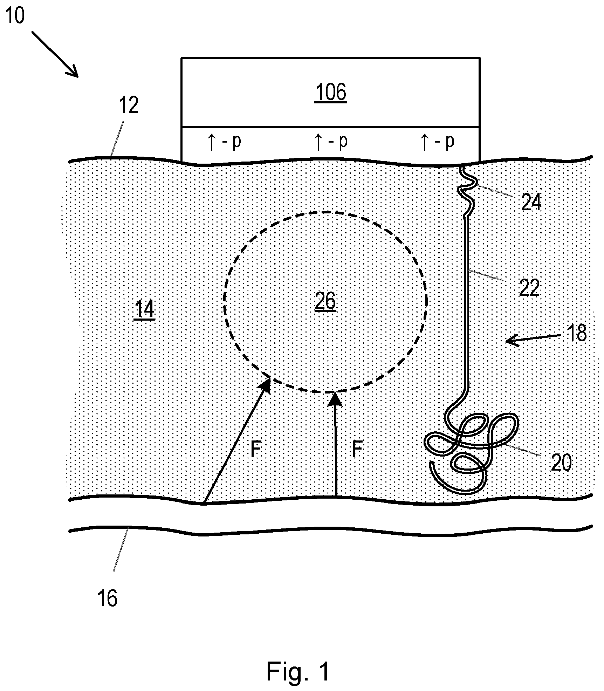

Referring to the drawings, Fig. 1 is a schematic illustration of part of a subject, showing a simplified version of various mechanisms taking place within the subject. The body part of the subject (e.g. an arm or a finger) is represented generally by reference numeral 10, and the body part includes skin 12 and tissues 14 under the skin. A capillary bed 16 transports blood through the subject. For clarity, a single capillary 16 is shown in Fig. 1. A sweat gland 18 includes a secretory coil 20, a dermal duct 22 and an upper coiled duct 24. The upper coiled duct 24 leads to an opening in the skin 12 of the subject, where sweat is secreted. Under normal conditions, as sweat generated in the secretory coil 20 travels up the dermal duct 22 towards the surface of the subject's skin 12, some of fluids may move from the dermal duct into the surrounding tissues 14 via reabsorption. Reabsorption is a biological process to prevent the secretion of useful components. For example, if the osmotic pressure within the tissues 14 is lower than the pressure of fluids in the dermal duct 22 of the sweat gland 18, then fluids may be caused to pass from the dermal duct into the surrounding tissues. Such reabsorption can affect the concentration of some biomarkers within the sweat, impacting on the correlation of the concentration of those biomarkers in sweat with the concentration of those biomarkers in the subject's blood.

According to embodiments disclosed herein, a pressure application unit 106 may be used to apply a negative pressure (-p) to the body part 10 of the subject at a region corresponding to and/or surrounding the sweat duct 18. The application of a negative pressure initially leads to a reduction in the osmotic pressure within the tissues 14, for example in the region 26 indicated by a dashed circle. To compensate for the reduced osmotic pressure, fluids move from the capillary bed 16 into the tissues in the region 26 (e.g. as indicated by arrows F), for example due to the relatively larger blood pressure in the capillary bed compared to the osmotic pressure in the region 26. Once a pressure equilibrium between the capillary bed 16 and the region 26 has once again been established, the osmotic pressure within the region 26 is relatively larger than it was prior to the application of the negative pressure. Due to the increased osmotic pressure in the region 26, reabsorption of fluids from the dermal duct 22 into the tissues in the region 26 is reduced, meaning more of the sweat generated at the secretory coil 20, and more of the biomarkers contained within the sweat, is secreted through the subject's skin 12. Consequently, the concentration of some biomarkers in the subject's sweat is more representative of (and correlates more closely to) the concentration of those biomarkers in the subject's blood.

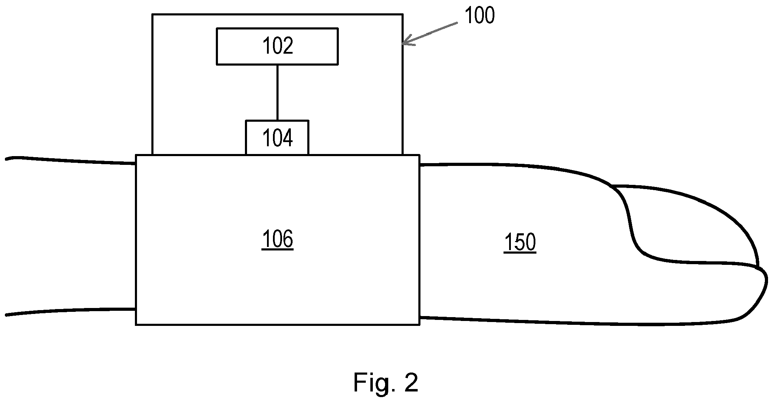

Fig. 2 is a schematic illustration of an example of an apparatus 100 for measuring a concentration of a biomarker in sweat generated by a subject. In this example, the apparatus 100 is shown attached to a finger 150 of the subject. However, in other embodiments, the apparatus 100 may be used with, and attached to, other parts of the subject's body.

The apparatus 100 comprises a processor 102, a biomarker sensor 104 and a pressure application unit 106. The biomarker sensor 104 is for receiving a volume of sweat secreted from a sweat gland of the subject and measuring a concentration of a biomarker in the volume of sweat. In use, the biomarker sensor 104 may be positioned such that it contacts the skin of the subject (e.g. skin 12 of the finger 150) to receive from pores in the skin. In other examples, sweat may be transported from the skin to the biomarker sensor 104 via a fluid transport mechanism, such as a microfluidic system. The biomarker sensor 104 may contain components (not shown) configured to detect one or more specific biomarkers (e.g. biomolecules having a particular characteristic and/or indicative of a particular condition) present in the volume of sweat. The biomarker measured using the biomarker sensor 104 may comprise any biomarker appropriate for determining information about the sweat and may, for example, comprise any biomarker discussed herein. In one specific example, the biomarker sensor 104 may be configured to measure a concentration of ions (e.g. sodium ions, potassium ions and/or chlorine ions), ethanol and/or cortisol in the sweat.

The pressure application unit 106 is configured to apply a negative pressure to the subject at the location of the sweat gland. As discussed in greater detail below, the pressure applied using the pressure application unit 106 may comprise a negative pressure or under-pressure (i.e. intended to initially reduce the osmotic pressure in tissue surrounding the sweat gland). In some embodiments, however, the pressure application unit 106 may also be configured to apply a positive pressure or overpressure (i.e. intended to increase the pressure in tissue surrounding the sweat gland). In the example shown, the pressure application unit 106 applies a negative pressure to the finger at the location of the sweat gland. It will be understood that the sweat gland is located under the subject's skin and that, by applying a negative pressure to the surface of the finger 150, pressure is initially reduced within the tissue under the skin and, accordingly, within the tissue surrounding the sweat gland (e.g. the tissue in the region 26 of Fig. 1). In embodiments in which the apparatus 100 is to be used with a different part of the subject's body, the pressure application unit 106 may be formed differently. For example, the pressure application unit 106 may be attached around an arm or a wrist of the subject.

The processor 102 is in communication with the biomarker sensor 104 and the pressure application unit 106. The processor 102 is configured to operate the pressure application unit 106 to apply a negative pressure to the subject at the location of the sweat gland. In some embodiments, the processor 102 may be configured to operate the pressure application unit 106 for a defined duration. Applying a negative pressure may initially cause a decrease in pressure in the interstitial fluid, but this may cause an increase in the amount of fluid caused to pass from the capillary bed 16 into the surrounding tissues. The amount of external pressure to be applied to the subject using the pressure application unit 106 may depend on the subject (e.g. on a pressure within the tissues and/or the sweat duct 18 of the subject). In general, the pressure application unit 106 may apply a negative pressure that meets or exceeds a defined pressure threshold at which reabsorption of fluid from a sweat duct of the sweat gland into surrounding tissues reduces. For example, the applied negative pressure may bring the hydrostatic pressure in the interstitial fluid to the threshold of 1 mm Hg. The estimation of hydrostatic pressure in the interstitial fluid is known to people skilled in the art, disclosed e.g. in: Himeno Y, Ikebuchi M, Maeda A, Noma A, Amano A. Mechanisms underlying the volume regulation of interstitial fluid by capillaries: a simulation study. Integr Med Res. 2016 Mar;5(1):11-21. In some examples, the applied negative pressure may be such that reabsorption of fluid from the sweat duct into the surrounding tissues reduces to below a defined threshold amount. In other words, the negative pressure applied using the pressure application unit 106 may be sufficient to prevent or substantially reduce reabsorption of fluids from the sweat duct (e.g. the dermal duct 22) into the surrounding tissues 26. Reabsorption may be considered to be sufficiently reduced if the amount of reabsorption is reduced to below the defined threshold amount.

As noted above, reabsorption of fluids from the dermal duct 22 into the surrounding tissues 26 may be significantly reduced or even stopped altogether if the pressure in the surrounding tissues 26 is equal to or greater than the pressure of fluids within the dermal duct 22. Thus, in some embodiments, the magnitude of the negative pressure applied using the pressure application unit 106 may be such that the applied negative pressure and an osmotic pressure within tissues 26 surrounding a sweat duct (e.g. the dermal duct 22) of the sweat gland 18 together exceed a pressure or fluids within the sweat duct of the sweat gland. For example, for a maximum pressure of fluids within the sweat duct around 70 kN/m$^2$ (~525 mm Hg) and osmotic pressure within tissues about 3 mm Hg, the maximum applied negative pressure may be 523 mm Hg. The pressure of fluids within the sweat duct may be known or estimated from literature e.g. Sonner Z, Wilder E, Heikenfeld J, Kasting G, Beyette F, Swaile D, Sherman F, Joyce J, Hagen J, Kelley-Loughnane N, Naik R. The microfluidics of the eccrine sweat gland, including biomarker partitioning, transport, and biosensing implications. Biomicrofluidics. 2015 May 15;9(3):031301.

The processor 102 is further configured to operate the biomarker sensor 104 to measure a concentration of the biomarker in a volume of sweat secreted from the sweat gland while the pressure application unit 106 is actively applying a negative pressure to the subject. In other words, after or during the pressure application, the biomarker sensor 104 measures the concentration of the biomarker in sweat secreted from the sweat gland. After the negative pressure has been applied to the subject by the pressure application unit 106 for a short time, the hydrostatic pressure of the interstitial fluid is increased due to the movement of fluid from the capillary bed 16 into the tissues 26, and transport of biomarker-containing fluid from the dermal duct 22 of the sweat gland 18 to the tissues 26 is reduced.

While, in the embodiment shown in Fig. 2, the processor 102 forms part of the apparatus 100 along with the biomarker sensor 104 and the pressure application unit 106, it will be appreciated that, in other embodiments, the processor 102 may be located remotely with respect to the biomarker sensor and/or the pressure application unit. For example, separate apparatuses may each include one of the biomarker sensor 104 and the pressure application unit 106, and these may each communicate with a remote processor 102 via a wired or wireless connection. For example, the processor 102 may form part of a computing device, such as a wearable device, a smart phone, a tablet computer, a laptop computer or a desktop computer, or may comprise a cloud-based processing facility.

The functions performed by the processor 102 described above may form part of a computer-implemented method, such as a computer-implemented method for measuring a concentration of a biomarker in sweat generated by a sweat gland of a subject. For example, such a computer-implemented method may be carried out using a processor and may comprise: operating a pressure application unit to apply a negative pressure to the subject at the location of the sweat gland for a defined duration; and operating a biomarker sensor to measure a concentration of the biomarker in a volume of sweat secreted from the sweat gland after or while the pressure application unit is actively applying a negative pressure to the subject.

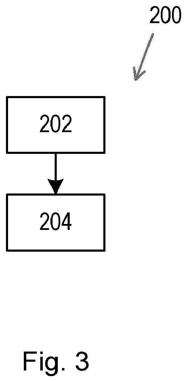

A more general method is discussed with reference to Fig. 3. Fig. 3 is a flowchart of an example of a method 200 for measuring a concentration of a biomarker in sweat generated by a subject. The method 200 comprises, at step 202, applying a negative pressure to the subject at the location of the sweat gland. In some embodiments, the external pressure may be applied for a defined duration. The negative pressure may be applied using the pressure application unit 106 discussed herein, and the pressure application unit may, in some embodiments, also be configured to apply a positive pressure. A negative pressure may be applied to a surface of the subject at the location of the sweat gland, so as to initially decrease a pressure (e.g. osmotic pressure) in tissues (e.g. interstitial fluid) around the sweat gland. The initially reduced pressure may be compensated for by movement of fluids from the capillary bed, leading to an overall increased pressure in the tissues surrounding the sweat gland, as described above. For example, a negative pressure may be applied to the subject using techniques disclosed herein.

The method 200 comprises, at step 204, measuring, after or while the negative pressure being applied for a defined duration, a concentration of the biomarker in sweat secreted from the sweat gland of the subject. The duration may for example be at least 1 minute, preferably at least 5 minutes, and more preferably at least 8 minutes, e.g. in the order of 10 minutes or it can be chosen according to the teachings of state of the art, e.g. Himeno et al. (2016). The pressure may be applied to the subject for a defined duration which is intended to be sufficient to allow an exchange of fluids within the subject's body to adapt to the applied pressure. As noted above, the application of a negative pressure ultimately increases the osmotic pressure in tissues 26 (e.g. the interstitial fluid) surrounding the sweat gland, thereby reducing the reabsorption of fluid from the sweat gland (e.g. the dermal duct of the sweat gland) into the tissues. By allowing a defined duration to elapse with the negative pressure being applied before measuring the concentration of the biomarker in the sweat, the change in the fluid transport within the subject's body is a given time to adapt before the concentration of the biomarker is measured. Various biomarkers may be detectable in sweat, and the particular biomarker whose concentration is to be measured varies from case to case. In some embodiments, a concentration of lactate may be measured in the sweat while, in other embodiments, a concentration of various ions (e.g. sodium ions, chlorine ions and potassium ions) may be measured in the sweat. In other embodiments, the concentration of other biomarkers may be measured or determined.

For some biomarkers, application of an external pressure (e.g. a negative pressure or a positive pressure) on the subject not only affects the movement of the biomarker between the capillary bed and the sweat glands into the surrounding tissues but, over time, may also reduce the influx of oxygen into cells of the sweat glands. This oxygen deprivation can bring about a change in the sweat gland cells, whereby the cells eventually switch to an anaerobic metabolism process, which in turn leads to an increased concentration of some biomarkers in sweat after a prolonged duration. Consequently, the measured concentration of some biomarkers may even increase due to the switch to anaerobic metabolism, rather than decreasing due to the application of external pressure.

To take account of and compensate for this effect, the method may, in some embodiments, make use of calibration values that can be generated *a priori*. For example, a curve may be rendered showing the concentration of the biomarker (e.g. lactate) in sweat as a function of average sweat rate per gland). The calibration values (e.g. the curve) may be used to estimate the rate of change of lactate concentration as function of the sweat rate.

In some embodiments, such a calibration may be performed in respect of the subject whose sweat is being analysed. For example, before the negative pressure is applied to subject, the sweat rate of the subject may be measured. Following the application of the negative pressure, the sweat rate may be measured again. A rate of change of the sweat rate may be used, as noted above, to calculate concentration of the biomarker.

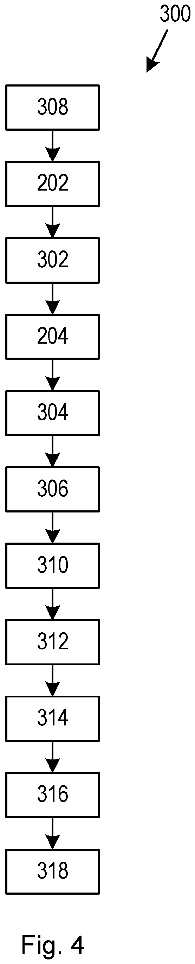

Fig. 4 is a flowchart of a further example of a method 300 for measuring a concentration of a biomarker in sweat generated by subject. The method 300 may comprise steps of the method 200 discussed above. The calibration steps discussed above may be performed as part of the method 300. In some embodiments, the method 300 may comprise, at step 302, measuring, prior to applying the negative pressure to the subject (step 202), a first sweat rate of the subject. At step 304, the method 300 may comprise measuring, following the application of the negative pressure, a second sweat rate of the subject. For example, the second sweat rate may be measured while the external pressure is being applied, after the defined duration has elapsed so as to allow the internal pressure of the subject's body to adjust. The method 300 may further comprise, at step 306, calculating a concentration of the biomarker in sweat secreted from the sweat gland of the subject based on the measured concentration and on the measured first sweat rate and the measured second sweat rate.

It is noted above that the negative pressure applied to the subject is intended to be sufficient to cause an overall increase in the pressure within the tissues 26 surrounding the sweat gland 18, in order to reduce the amount of reabsorption of fluids from the sweat gland into the surrounding tissues. The pressure of fluids within the sweat gland 18 (e.g. within the dermal duct 22 of the sweat gland) as it has already been discussed may be known from literature. For example, the pressure of fluids within the sweat glands of a particular type of person may be recorded in scientific papers, and the negative pressure applied to the subject may be selected so as to ensure that the total pressure (i.e. the osmotic pressure combined with the effect of the applied negative pressure) exceeds the pressure of fluids within the sweat glands. In other examples, however, the pressure of fluids within the sweat gland may be measured, for example using a pressure measurement device. In such examples, the negative pressure applied to the subject (e.g. a magnitude of the applied negative pressure) may be based on the measured pressure of fluids within the sweat gland. Thus, the method 300 may further comprise, at step 308, determining a pressure of fluids within a sweat duct of the sweat gland 18. As noted above, determining the pressure may comprise obtaining a known or reference pressure, for example from literature, or measuring the pressure using a measurement device. The negative pressure applied to the subject (step 202 may be based on the determined pressure.

As noted above, the pressure applied to the subject is a negative pressure (e.g. an under pressure caused by suction). In one embodiment, the method 300 may further comprise, at step 310, applying, after measuring the concentration of the biomarker (step 204), a positive pressure to the subject at the location of the sweat gland, so as to increase a pressure in interstitial fluid around the sweat gland. In some embodiments, as discussed below, it may be possible to apply a positive pressure to the subject using the same device or unit (e.g. the pressure application unit 106) used to apply a negative pressure to the subject. In this way, the benefits of applying both a positive pressure and a negative pressure can be realised in rapid succession. By applying a positive pressure, different mechanisms occurring within the subject's body may be overcome or compensated for, and this can be beneficial for measuring some biomarker concentrations in sweat.

Once the negative pressure has been applied to the subject, the subject's body is typically relatively quick to react and take action to equilibrate the pressure within the tissues, for example by moving fluid from the capillary bed 16 into the tissues 26 surrounding the sweat gland 18, where the pressure has temporarily been reduced. Once a pressure equilibrium within the tissues 26 has been reached, if the application of the negative pressure is removed, the body of the subject can be relatively slower to adjust back to the previous pressure equilibrium, by re-absorbing fluid back into the capillary bed 16 from the surrounding tissues 26. This means that the negative pressure can be applied for a relatively short time, and the effects on the pressure within the subject's body remain for substantially longer, so that measurements of the concentration of the biomarker in the subject's sweat can be taken even after the application of the negative pressure has been removed, and the correlation between the biomarker concentration in sweat and the biomarker concentration in the subject's blood remains. Thus, the method 300 may further comprise, at step 312, removing, after the expiry of the defined duration, the application of the negative pressure. The defined duration may be selected based on the time taken for a pressure equilibrium within the subject to be reached.

In some examples, removal of the application of the negative pressure (i.e. step 312) may be performed prior to measuring the concentration of the biomarker, at step 204.

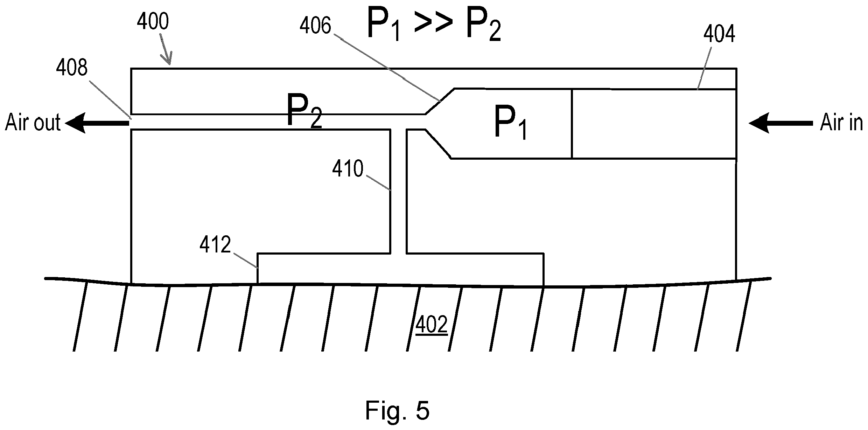

Several different techniques may be used to apply a negative pressure to the skin of a subject. Fig. 5 is a schematic illustration of an example of one technique by which a negative pressure may be applied. The technique shown in Fig. 5 utilises the Venturi effect, or the Bernoulli principle. In the example shown in Fig. 5, a device 400, which may include a sweat sensor (not shown) is positioned on skin of a subject 402. The device 400 includes a compressor or fan 404 to blow air or fluid (e.g. old sweat) at high speed (e.g. between 10 and 100 m/s) through a converging channel 406 towards an outlet 408. The channel 406 is connected to a further channel 410, leading to a chamber 412 formed between the device and the skin of the subject 402. As the air or fluid is pumped through the converging channel 406, an under-pressure (e.g. a partial vacuum) is formed in the chamber 412. The formation of this under-pressure is governed by Bernoulli's equation:

$$P_0 = P_1 + 0.5\rho_1 U_1^2 = P_2 + 0.5\rho_2 U_2^2 \qquad [2]$$

where $P_0$ is the total pressure of a system, $P_1$ is the pressure at a first position in the system, $P_2$ is the pressure at a second position in the system, $\rho_1$ is the fluid density at the first position, $\rho_2$ is the is the fluid density at the second position, $U_1$ is the velocity of the air or fluid at the first position in the system, and $U_2$ is the velocity of the air or fluid at the second position in the system.

Assuming incompressible flow implies $\rho_1 = \rho_2 = \rho$, and given $U_1 \approx 0$, this yields:

$$P_1 = P_2 + 0.5\rho U_2^2 \qquad [3]$$

where $P_2 \ll P_1$. Regions having the pressures $P_1$ and $P_2$ are shown in Fig. 5.

The compressor or fan can be activated when sweat sampling is required. However, as sweat is produced and/or as a seal between the device 400 and the skin of the subject 402 begins to leak, the under-pressure will slowly reduce. If there is a requirement to maintain the under-pressure for a longer period of time, it may be necessary to re-activate the compressor or fan.

In other embodiments, an active pump may be used to create a reduced pressure, or used/wasted sweat may be used to drive a turbo motor in order to draw more sweat into the device.

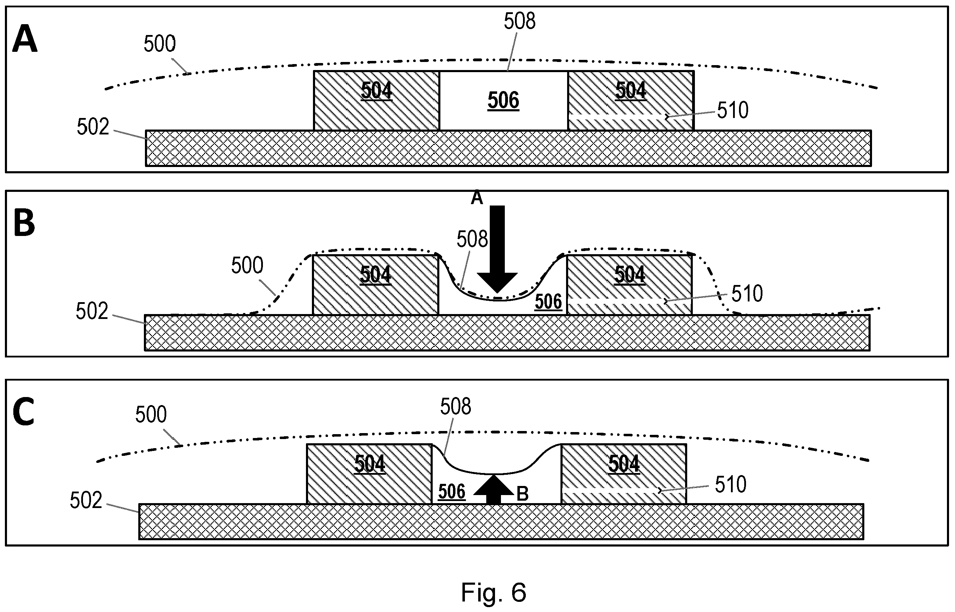

In an alternative embodiment, a device used for creating a positive pressure may also be used for creating a negative pressure. Fig. 6 is a schematic cross-sectional illustration of an arrangement in which a device 500 capable of creating a positive pressure is used to generate a negative pressure, or under-pressure. In this example, the device 500 comprises a cuff of a device used for measuring a subject's blood pressure. A substrate 504 is positioned between the cuff 500 and the subject's skin 502, such that the walls of the substrate form sidewalls of an enclosed chamber 506. For example, the substrate 504 may comprise a ring-shaped or annular structure. A flexible membrane 508 positioned between opposing sides of the substrate 504 forms a top wall of the chamber 506, and the skin of the subject 502 forms the bottom wall of the chamber. In some examples, the substrate 504 may comprise part of a wearable device or patch that includes a sweat sensor. A vent 510 is formed through the substrate 504 and is configured to allow the passage of fluid from the chamber 506 to the ambient air surrounding the substrate. A one-way valve may be provided to allow air out of the device through the vent 510, but preventing the flow of air into the device through the vent. In Fig. 6A, the cuff 500 is not inflated, so it is not applying a positive pressure to skin of the subject 502. Fig. 6B shows the arrangement when the cuff 500 is inflated, so as to apply a positive pressure to the subject 502. Inflation of the cuff 500 urges the flexible membrane downwards towards the skin of the subject 502, forcing air within the chamber 506 out through the vent 510, and generating a positive, downward pressure on the subject, as indicated by the arrow A. Fig. 6C shows the arrangement when the cuff 500 has deflated, such that the cuff no longer applies a positive pressure on the subject 502. Since air is unable to return through the vent 510 into the chamber 506, when the flexible membrane 508 tries to return to its original form (as shown in Fig. 6A), it creates a negative pressure (i.e. an under-pressure) within the cavity, as indicated by the arrow B. Thus, a standard "off-the-shelf" blood pressure cuff can be used to generate both a positive pressure and a negative pressure.

The application of a negative pressure to the subject can be particularly advantageous when measuring the concentration of some biomarkers. The creation of a negative pressure causes ions in fluid to be forced out the subject's bloodstream (i.e. capillaries) into the interstitial fluid, and also into the sweat glands. Thus, the sweat glands have an increased concentration of ions, but it can be assumed that, due to the negative pressure, these are from the blood. Since the concentration of ions in the interstitial fluid is high, the reabsorption of the ions from the sweat glands and from the sweat ducts, that connect the glands to the surface of skin, into the interstitial fluid is greatly reduced. Therefore, the concentration of the ions (i.e. the biomarker) in sweat measured while a negative pressure is applied is approximately equal to the concentration of the biomarker in the blood of the subject. The same is true of other biomarkers, including for example ethanol and cortisol.

Filtration from capillaries is also driven by the osmotic/oncotic pressure, as shown in equation [1] above. A normal oncotic pressure (e.g. for a healthy person) in plasma (referred to as $n_c$) is around 25 mmHg and a normal oncotic pressure of interstitial fluid (referred to as $\pi_i$) is around 3 mmHg. The main contributors to oncotic pressure in plasma are plasma proteins (mainly albumin). Since only $n_i$ contributes to the hydrostatic blood pressure $P_c$, it is a relatively small component and may, in some embodiments, be either added to an external positive pressure applied or, in other embodiments, it may be assumed that the effect is negligible.

In some embodiments, it may be advantageous to measure the concentration of a biomarker repeatedly over a period of time. In some examples, biomarker concentration measurement may be taken repeatedly during the application of the negative pressure, for example while the magnitude of the negative pressure applied by the pressure application unit 106 is increasing and/or decreasing.

Measurements of biomarker concentrations taken during a ramped inflation and/or a ramped deflation of the pressure application unit (e.g. a cuff) over a period of time may be used to generate a calibration curve for the biomarker of interest. Thus, in some embodiments, the method 300 may comprise, at step 314, measuring, during the application of the negative pressure (step 202), a plurality of concentrations of the biomarker. The method 300 may further comprise, at step 316, calculating a concentration of the biomarker in sweat secreted from the sweat gland of the subject based on the plurality of measurements. For example, a calibration curve may be generated based on the measured concentrations of the biomarker over time, for example as the magnitude of the applied negative pressure increases.

In some embodiments, pressure ramping may be performed in a manner similar to that used when taking blood pressure measurements using a blood pressure measurement cuff. The magnitude of the applied negative pressure may be increased, and then decreased in discrete steps. At each step, biomarker concentration measurements are taken over a period of time (e.g. in the order of minutes to tens of minutes) to enable a calibration curve between the applied pressure and the biomarker concentration to be determined.

It may be intended that the step interval is long enough to permit changes in pressure in the tissues 26 and surrounding the sweat gland 18. Such a calibration step may be performed intermittently (e.g. daily, weekly or monthly) to account for changes in patient metabolism over time which may be influenced by medication administration, disease progression and fitness. Thus, the calibration may be considered to be a re-calibration.

Thus, with reference again to Fig. 4, the step of applying the external pressure (step 202) may comprise decreasing the magnitude of the applied negative pressure in regular steps, and measuring, at each step, a concentration of the biomarker in sweat secreted from the sweat gland of the subject. The method 300 may further comprise, at step 316, generating a calibration curve using the measured concentrations. The method 300 may further comprise, at step 318, calculating a concentration of the biomarker in sweat based on the generated calibration curve. In other words, the calibration may be used to calculate the concentration of the biomarker.

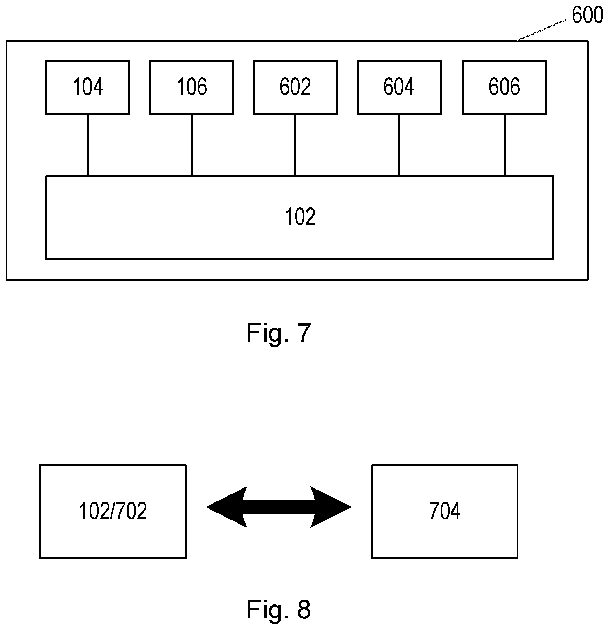

Fig. 7 is a schematic illustration of a further example of an apparatus 600 for measuring a concentration of a biomarker in sweat generated by subject. The apparatus 600 may comprise, or be similar to, the apparatus 100 discussed above. Specifically, the apparatus 600 comprises a biomarker sensor 104, a pressure application unit 106 and a processor 102 in communication with the biomarker sensor and the pressure application unit. The processor 102 is configured to perform the functions discussed above in connection with Fig. 2.

As discussed above, the pressure application unit 106 may comprise a unit configured to apply a negative pressure on the subject. In some examples the pressure application unit 106 may also apply a positive pressure on the subject. In some embodiments, the pressure application unit 106 may be configured to apply both a positive pressure and a negative pressure, either successively or concurrently, using the techniques discussed herein.

The processor 102 is, in some embodiments, configured to operate the pressure application unit 106 to apply a negative pressure having a magnitude that meets or exceeds a defined pressure threshold at which reabsorption of fluid from a sweat duct of the sweat gland into surrounding tissues reduces. In some examples, the applied negative pressure may be such that reabsorption of fluid from the sweat duct into the surrounding tissues reduces to below a defined threshold amount. In other words, the negative pressure applied by the pressure application unit 106 may be such that reabsorption of fluids into tissues from the sweat duct is effectively prevented. This may be achieved by controlling magnitude of the applied negative pressure to be large enough that, together with the osmotic pressure in the tissues surrounding the sweat duct, the combined pressure is greater than the pressure of fluids within the sweat duct itself.

In some embodiments, the biomarker sensor 104 may be configured to measure the concentration, in the volume of sweat, of a biomarker selected from a group comprising: lactate, sodium ions, potassium ions, chlorine ions, urea, ethanol, ammonium, glucose and cortisol.

The apparatus 600 may, in some embodiments, further comprise a pressure measurement unit 602 for measuring a pressure of fluids within a sweat duct of the sweat gland. The pressure measurement unit 602 may be in communication with the processor 102. The processor 102 may be configured to operate the pressure application unit 106 to apply a negative pressure based on the measured pressure. For example, the processor 102 may be configured to operate the pressure application unit 106 to apply a negative pressure that equals or exceeds the measured pressure.

The apparatus 600 may, in some embodiments, further comprise a timer 604 in communication with the processor 102, and configured to measure at least one of: a duration of application of the applied negative pressure; and the timing of measurements of the biomarker concentrations.

Fig. 8 is a schematic illustration of an example of a processor 702 in communication with a computer-readable medium 704. The processor 702 may comprise or be similar to the processor 102 discussed above. According to some embodiments, a computer program product comprises a non-transitory computer-readable medium 704, the computer-readable medium having computer-readable code embodied therein, the computer-readable code being configured such that, on execution by a suitable processor 102, 702, the processor is caused to operate the pressure application unit to apply a negative pressure to the subject at the location of the sweat gland; operate a biomarker sensor to measure a concentration of the biomarker in a volume of sweat secreted from the sweat gland after or while the pressure application unit has actively applied a negative pressure to the subject for a defined duration. In other embodiments, the computer-readable medium may comprise code configured such that, on execution, the processor is further caused to perform one or more of the steps or functions discussed herein.

The processor 102, 702 can comprise one or more processors, processing units, multicore processors or modules that are configured or programmed to control the apparatus 100, 600 in the manner described herein. In particular implementations, the processor 102, 702 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. Measures recited in mutually different dependent claims can be advantageously combined. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A method (200) for measuring a concentration of a biomarker in sweat generated by a subject, the method comprising:

   applying (202) a negative pressure to the subject at the location of the sweat gland;
   measuring (204) a concentration of the biomarker in sweat secreted from the sweat gland of the subject, after the negative pressure has being applied, or while the negative pressure is applied, for a defined duration.

2. A method (200) according to claim 1, wherein the applied negative pressure meets or exceeds a defined pressure threshold at which reabsorption of fluid from a sweat duct of the sweat gland into surrounding tissues reduces.

3. A method (200) according to claim 1, wherein applying a negative pressure comprises applying a pressure having a magnitude such that the applied negative pressure and an osmotic pressure within tissues surrounding a sweat duct of the sweat gland together exceed a pressure of fluids within the sweat duct of the sweat gland.

4. A method (200, 300) according to any of the preceding claims, further comprising:

   determining a pressure of fluids within a sweat duct of the sweat gland;
   wherein the negative pressure applied to the subject is based on the determined pressure.

5. A method (200, 400) according to any of the preceding claims, further comprising:

   measuring (302), prior to applying the negative pressure to the subject, a first sweat rate of the subject;
   measuring (404), following the application of the negative pressure, a second sweat rate of the subject; and
   calculating a concentration of the biomarker in sweat secreted from the sweat gland of the subject based on the measured concentration and on the measured first sweat rate and the measured second sweat rate.

6. A method (200, 300) according to any of the preceding claims, further comprising:
   applying (310), after measuring the concentration of the biomarker, a positive pressure to the subject at the location of the sweat gland, so as to increase a pressure in interstitial fluid around the sweat gland.

7. A method (200, 300) according to any of the preceding claims, further comprising:

   measuring (314), during the application of the negative pressure, a plurality of concentrations of the biomarker; and
   calculating (316) a concentration of the biomarker in sweat secreted from the sweat gland of the subject based on the plurality of measurements.

8. A method (200, 300) according to any of the preceding claims, wherein applying the negative pressure comprises:

   decreasing a magnitude of the applied negative pressure in regular steps; and
   measuring, at each step, a concentration of the biomarker in sweat secreted from the sweat gland of the subject;
   wherein the method further comprises:

      generating (316) a calibration curve using the measured concentrations; and
      calculating a concentration of the biomarker in sweat based on the generated calibration curve.

9. A method (200, 300) according to any of the preceding claims, further comprising:
   removing (312), after the expiry of the defined duration, the application of the negative pressure.

10. An apparatus (100, 600) for measuring a concentration of a biomarker in sweat generated by a subject, the apparatus comprising:

    a biomarker sensor (104) for receiving a volume of sweat secreted from a sweat gland of the subject and measuring a concentration of a biomarker in the volume of sweat;
    a pressure application unit (106) configured to apply a negative pressure to the subject at the location of the sweat gland; and
    a processor (102) in communication with the biomarker sensor and the pressure application unit, and configured to:

operate the pressure application unit to apply a negative pressure to the subject at the location of the sweat gland;

operate the biomarker sensor to measure a concentration of the biomarker in a volume of sweat secreted from the sweat gland after the pressure application unit has applied, or while the pressure application unit applies a negative pressure to the subject, for a defined period.

11. An apparatus (100, 600) according to claim 10, wherein the processor is configured to operate the pressure application unit to apply a negative pressure having a magnitude that meets or exceeds a defined pressure threshold at which reabsorption of fluid from a sweat duct of the sweat gland into surrounding tissues reduces.

12. An apparatus (100, 600) according to claim 10 or claim 11, further comprising:

a pressure measurement unit (602) for measuring a pressure of fluids within a sweat duct of the sweat gland; wherein the processor is configured to operate the pressure application unit to apply a negative pressure based on the measured pressure.

13. An apparatus (100, 600) according to any to claims 10 to 12, further comprising:

a timer (604) in communication with the processor, and configured to measure at least one of: a duration of application of the applied negative pressure; and the timing of measurements of the biomarker concentrations.

14. An apparatus (100, 800) according to any to claims 10 to 13, wherein the biomarker sensor is configured to measure the concentration, in the volume of sweat, of a biomarker selected from a group comprising: lactate, sodium ions, potassium ions, chlorine ions, urea, ethanol, ammonium, glucose and cortisol.

15. A computer program product comprising computer-readable code configured such that, on execution by a processor (102, 702), the processor (102, 702) is caused to:

operate the pressure application unit to apply a negative pressure to the subject at the location of the sweat gland; and

operate a biomarker sensor to measure a concentration of the biomarker in a volume of sweat secreted from the sweat gland after the pressure application unit has applied, or while the pressure application unit applies a negative pressure to the subject, for a defined period.

Fig. 1

EP 4 085 836 A1

Fig. 2

200

```
┌─────────────┐
│     202     │
└─────────────┘
       │
       ▼
┌─────────────┐
│     204     │
└─────────────┘
```

Fig. 3

300

Fig. 4

Fig. 5

Fig. 6

Fig. 7

Fig. 8

Europäisches
Patentamt

European
Patent Office

Office européen
des brevets

# EUROPEAN SEARCH REPORT

Application Number

EP 21 17 2330

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/196760 A1 (HYDE RODERICK A [US] ET AL) 13 July 2017 (2017-07-13) * paragraphs [0103] - [0106], [0133], [0158] - [0160]; figure 5 * | 1-3,9, 11,13,14 | INV. A61B5/145 |
| A | WO 2018/006087 A1 (UNIV CINCINNATI [US]; ECCRINE SYSTEMS INC [US]) 4 January 2018 (2018-01-04) * paragraphs [0035], [0042] * | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 26 October 2021 | Knüpling, Moritz |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.........................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 17 2330

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2021

| Patent document cited in search report | Publication date | Patent family member(s) | Publication date |
|---|---|---|---|
| US 2017196760 A1 | 13-07-2017 | US 2017196760 A1<br>US 2017196761 A1<br>US 2021106493 A1 | 13-07-2017<br>13-07-2017<br>15-04-2021 |
| WO 2018006087 A1 | 04-01-2018 | EP 3478186 A1<br>US 2019175094 A1<br>WO 2018006087 A1 | 08-05-2019<br>13-06-2019<br>04-01-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HIMENO Y ; IKEBUCHI M ; MAEDA A ; NOMA A ; AMANO A.** Mechanisms underlying the volume regulation of interstitial fluid by capillaries: a simulation study. *Integr Med Res.,* March 2016, vol. 5 (1), 11-21

- **SONNER Z ; WILDER E ; HEIKENFELD J ; KASTING G ; BEYETTE F ; SWAILE D ; SHERMAN F ; JOYCE J ; HAGEN J ; KELLEY-LOUGHNANE N.** The microfluidics of the eccrine sweat gland, including biomarker partitioning, transport, and biosensing implications. *Biomicrofluidics,* 15 May 2015, vol. 9 (3), 031301